# EUROPEAN PATENT SPECIFICATION

(11) **EP 0 420 620 B1**
(45) Date of publication and mention of the grant of the patent: **28.06.1995**
(21) Application number: 90310550.0
(22) Date of filing: 26.09.1990
(51) Int. Cl.: A61M 5/142

(54) **Pressure regulator for implantable pump**
Druckregler für eine implantierbare Pumpe
Régulateur de pression pour pompe implantable

(30) Priority: 26.09.1989 US 412376
(43) Date of publication of application: 03.04.1991
(73) Proprietor: INFUSAID INC., Norwood, Massachusetts (US)
(72) Inventor: Burke, Paul, Woonsocket, Rhode Island (US)
(74) Representative: Couchman, Jonathan Hugh

(56) References cited:
- EP-A- 0 039 124
- WO-A-80/02377

## Description

This invention is directed to a system for accurately controlling a flow rate of a drug from an implantable drug delivery device and maintaining in-vivo a higher degree of safety. Devices of this type have reached the point of commercial utilization as typified by the Infusaid models 100 and 400 devices available from Infusaid Inc., Norwood, Massachusetts USA. Such devices are implantable to deliver a drug at a very slow flow rate over a long period of time. They are refilled subcutaneously.

An implantable infusion pump of the prior art (US 3,731,681) utilizes the vapour pressure of a two stage gas to provide a constant pressure for a drug flowing through a capillary tube in order to maintain a constant flow rate. This technique of flow control, while simple and reliable, is sensitive to outside variables such as changes in temperature and atmospheric pressure. For example, if the patient has a fever, or works in a cold environment, the temperature of the implanted gland can change several degrees. The internal pressure change is about 157.6 N/m per _{°} C (0.5 psi per degree F). A 25 percent increase in pressure and drug flow rate can result from a fever of 39.2 °C (102.5 F). This change is more than can be tolerated when a critical drug is being administered. A more serious situation results from the reduced air pressure in airplane cabins when the patient is travelling. The standard airplane pressure is maintained at a level corresponding to an altitude of about 1524m (5,000 feet) above sea level. With a gland using a 1436 N/m (8.2 psi) internal pressure, this would increase the differential pressure by 26 percent over the sea level setting. Although the drug dosage can be adjusted by changing the concentration of drug in the gland, this is a serious inconvenience and hardship for patients who must travel by air frequently.

To compensate for variations in temperature and pressure, another implantable pump of the prior art (US 4,299,220) uses a flow regulator to compensate for variations in temperature and pressure. This regulating device comprises a pair of chambers separated by a flexible diaphragm. The first chamber is in fluid contact with the reservoir of the pump and has an outlet allowing the reservoir fluid to flow into a restrictor. This restrictor flows into the other chamber and communicates with the pump outlet via a centrally located regulating seal. The relationship of the regulator seal to the flexible diaphragm permits the regulator to maintain a constant pressure drop across the restrictor despite reservoir or outlet pressure variations.

In more detail, the flow regulator comprises a body made up of mating top member and bottom member. The top portion of the bottom body member has a deep recess into which the bottom portion of top body member is received in mating relation. A resilient gasket is seated in the cavity bottom. A resilient diaphragm composed of flexible but impervious material (such as 0.076 mm - 0.003 inch - titanium metal, for example) is disposed in the cavity resting on the resilient gasket. A resilient O-ring is disposed in the cavity on top of the diaphragm so as to engage the bottom chamfered edge of the top member so as to seal the unit when the top and bottom members are assembled and fastened together, as by means of a plurality of screws. Alternatively, the upper and lower body members and diaphragm may be welded together into an integral unit. A shallow recess in the bottom surface of the top member along with the remaining space in the bottom body portion after assembly of the body, forms a shallow internal cavity which is divided by the diaphragm into a first or upper chamber and a second or lower chamber. Ordinarily there are opposing forces on the diaphragm which balance out and the diaphragm is stationary. If the forces are unbalanced, for example, by a decrease in flow that reduces the pressure difference across the capillary, the diaphragm will deflect. The diaphragm position determines the resistance to flow of the control valve. The control valve seal presses against the low pressure side of the diaphragm and, therefore, will open the valve when the pressure drop across the capillary is low and close the valve when the pressure drop is high. This negative feedback controls the flow to maintain it constant at a value determined by the balance of pressure on the diaphragm. Flow rates as small as 1 ml/day may be controlled to within ± 5 percent. This flow rate is so low that even small leakage paths through the valve would exceed the control range. The polished surface of the diaphragm forces an elastomer O-ring to conform to its surface profile to reduce the leakage path to zero when fully seated.

While this configuration provides pressure regulation, the flow paths have potentially undesirable failure modes. For example, since the regulator depends on the drug fluid to supply the regulator sensing pressure, a diaphragm failure, such as a hole in the membrane, would allow the drug to bypass the regulator's restrictor resulting in an undesirable increase in flow rate. Another potential problem is a failure of the regulator seal, that is, a seal leak or physical dislocation of the seal relative to the seat. This would result in a flow rate increase due to the lack of seal restriction.

According to the present invention there is provided an implantable drug delivery system comprising a pressure actuable drug dispensing device having a sealed housing, a flexible drug reservoir containing a fluid, an outlet from said drug reservoir and a propellant chamber to urge said fluid from said reservoir into said outlet in use of the system, a flow regulating device having a regulator chamber and a sensing chamber, said regulator chamber and said sensing chamber being separated and maintained in isolation from each other by a flexible diaphragm, said outlet from said drug reservoir being in fluid communication with said flow regulating device and said regulating device having an outlet catheter which couples said regulator chamber in use to a drug delivery site, characterized in that said outlet can supply said fluid only to said regulator chamber, and means is provided to establish a reference pressure in said sensing chamber that is substantially equal to the pressure in said propellant chamber.

In another aspect, there is provided a drug delivery system for delivering a drug-containing liquid to a drug delivery site, which system comprises pressure-operable supply means comprising means defining a compressible reservoir for containing a charge of said liquid and a pressure medium-containing enclosure disposed for applying said pressure medium to said compressible reservoir-defining means to drive drug-containing liquid from said reservoir in use, drug-containing liquid flow regulation means for receiving said liquid, connection means providing communication for liquid flow between said reservoir and said flow regulation means, and outlet means for transmitting drug-containing liquid in use from said flow regulation means to said delivery site, said flow regulation means comprising first and second enclosures isolated one from the other by means of a flexible liquid-impervious diaphragm displaceable in response to pressure differential between said first and second enclosures to control liquid flow rate into said outlet means from said flow regulation means, characterized in that said connection means and said outlet means communicate only with said first flow regulation means enclosure and means is provided to establish in said second flow regulation means enclosure a pressure active to displace said diaphragm and variable responsively to causes of variation in the instantaneous pressure of said pressure medium in said pressure-operable supply means so that said pressures are substantially matched and the diaphragm is displaced to provide stabilized rate of delivery of said drug-containing liquid.

The invention will now be described by way of example only, reference being made to the accompanying drawings in which:-
Figure 1 is a first embodiment of an implantable drug system in accordance with this invention;
Figure 2 is a second embodiment of this invention utilizing a downstream restrictor element in accordance with this invention;
Figure 3 is a third embodiment of this invention which couples the propellant and sensing chambers together in accordance with this invention; and
Figure 4 is a fourth embodiment of this invention utilizing the combination of a downstream restrictor and the propellant and sensing chambers coupled together.

Referring now to Figure 1, a first preferred embodiment of this invention is illustrated in which a drug delivery device comprises an implantable infusion pump 10 having a housing 12 divided into a drug reservoir chamber 14 and a propellant chamber 16 by a bellows diaphragm 18. A septum 20 is formed of a penetrable resilient stopper material and provides a fluid communication to allow for subcutaneous refilling of the drug reservoir 14. The propellant chamber 16 contains a liquid such as "Freon" having a vapor pressure which, under condition of normal body pressure exerts a pressure on the bellows to force a drug contained in the drug reservoir 14 through outlet conduit 22. A bacteria filter 24 can be placed in-line between the drug reservoir 14 and the outlet conduit 22. A flow restrictor 28 is placed on the outlet conduit 22 to provide a suitable pressure drop.

In accordance with this invention a flow regulating device 30 comprises a regulator chamber 32 and a sensing chamber 34 divided by a thin flexible diaphragm 36. A regulator seal 38 is employed to define a stop for the flexible diaphragm 36. The regulator seal 38 and the flexible diaphragm 36 are worked in the manner described earlier herein with reference to US Patent 4 299 220. A drug at the delivered pressure is then input into flow regulating device 30.

In the prior art device, a regulator was in fluid contact with the implantable infusion pump drug reservoir and maintained a constant flow rate by compensating for changes in the pump reservoir or outlet pressures. Thus, the prior art uses a diaphragm-to-seal technology which has the sensing chamber in fluid contact with the reservoir. However, in accordance with this invention, as illustrated in Figure 1, the sensing chamber 34 is sealed from the fluid flow path. It is filled with the same 2-phase fluid as in the pump propellant chamber 16. Thus, the sensing chamber 34 contains the same vapor pressure as the pump propellant chamber 16. It thus parallels pressure fluctuations experienced by the propellant chamber 16. Thus, the fact that the regulator contains an independent pressure source allows the sensing chamber 34 to operate without commnunicating with the drug reservoir 14. This eliminates the possibility of drug solution bypassing the regulator due to a diaphragm hermeticity failure.

The regulator of Figure 1 can be an independent component added to the outlet 22 of the pump system, as shown, or alternatively can be contained within the housing for the implantable pump component within the container 12.

Referring now to Figure 2, a second embodiment of this invention is illustrated which incorporates a second restrictor 40 to establish a pressure drop across the regulator 30. Thus, the first restrictor 26 establishes a pressure drop between the pump 10 and the regulator 30 while the downstream second restrictor 40 establishes a pressure drop between an outlet catheter 42 and the flow regulating device 30. The restrictor 26 has a low restrictive value, and is used to establish the pressure drop across the regulating chambers 32 and 34. However, the majority of the pump's flow restriction is established by the second restrictor 40 located downstream of the regulator seal. With the majority of the restriction located downstream, a failure in the regulator seal 38 will not result in excessive pump flow rates.

Referring now to Figure 3, a third embodiment of this invention is illustrated in which the propellant chamber 16 is physically coupled to the sensing chamber 34 in the regulating device 30 via a line 44. The embodiment of Figure 3 departs from that of Figure 1 in that, instead of having the sensing chamber 34 provided with an independent source of 2-phase propellant fluid, the same fluid is used in common in chamber 16 and chamber 34.

Figure 4 represents a fourth embodiment that is a combination of Figures 2 and 3. In Figure 4 the combination of upstream restrictor 26 and downstream second restrictor 40 is used to provide two differential pressure drops respectively between the pump 10 and the regulator 30 and the outlet catheter 42. Additionally, the common interconnection of the propellant chamber 16 and the sensing chamber 34 utilizing the line 44 is accomplished so that those two chambers are in direct fluid communication with each other.

As is apparent, in each of these embodiments regulator sensing is not dependent on the flow of drug from the outlet 22. Rather, in each embodiment the sensing chamber in the regulator is established with either an independent source of propellant to establish a base line regulating pressure or, is coupled in direct fluid communication with the propellant chamber in the drug dispensing device. In addition, by the use of a downstream restrictor, a failure in the regulator seal will not cause a catastrophic failure since the restrictor will still establish the necessary pressure drop.

## Claims

1. An implantable drug delivery system comprising a pressure actuable drug dispensing device (10) having a sealed housing (12), a flexible drug reservoir (14) containing a fluid, an outlet (22) from said drug reservoir (14) and a propellant chamber (16) to urge said fluid from said reservoir (14) into said outlet (22) in use of the system, a flow regulating device (30) having a regulator chamber (32) and a sensing chamber (34), said regulator chamber (32) and said sensing chamber (34) being separated and maintained in isolation from each other by a flexible diaphragm (36), said outlet (22) from said drug reservoir (14) being in fluid communication with said flow regulating device (30) and said regulating device (30) having an outlet catheter (42) which couples said regulator chamber in use to a drug delivery site, characterized in that said outlet (22) can supply said fluid only to said regulator chamber (32), and means is provided to establish a reference pressure in said sensing chamber (34) that is substantially equal to the pressure in said propellant chamber.

2. A system as claimed in claim 1 wherein said means to establish a reference pressure comprises a source of propellant in said sensing chamber (34) that is the same as the propellant in the propellant chamber (16) of said drug dispensing device (10).

3. A system as claimed in Claim 1 or Claim 2 wherein said means to establish a reference pressure comprises a fluid coupling (44) between said propellant chamber (16) and said sensing chamber (34).

4. A system as claimed in Claim 2 or Claim 3 and comprising a restrictor (40) in said outlet catheter (42).

5. A system as claimed in any preceding claim and comprising a penetration septum (20) coupled to said drug reservoir (14) for refilling said fluid.

6. A system as claimed in any preceding claim and comprising a bacterial filter (24) in said outlet conduit (22) and a flow restrictor (26) disposed in said outlet conduit upstream of said regulator chamber (32).

7. A system as claimed in any preceding claim and comprising a regulator seal (38) in said regulating chamber (32) of said flow regulating device (30) said regulator seal (38) being disposed relative to said diaphragm (36) to vary the flow of fluid to said outlet catheter (42).

8. A system as claimed in Claim 7 wherein said flexible diaphragm (36) is biased to deflect in the direction of said regulator seal (38).

9. A system as claimed in any preceding claim wherein said flexible drug reservoir (14) comprises a bellows (18) disposed in said sealed housing (12).

10. An implantable drug delivery system for delivering a drug-containing liquid to a drug delivery site, which system comprises pressure-operable supply means (10) comprising means defining a compressible reservoir (14) for containing a charge of said liquid and a pressure medium-containing enclosure (16) disposed for applying said pressure medium to said compressible reservoir-defining means (14) to drive drug-containing liquid from said reservoir (14) in use, drug-containing liquid flow regulation means (30) for receiving said liquid, connection means (22) providing communication for liquid flow between said reservoir (14) and said flow regulation means (30), and outlet means (42) for transmitting drug-containing liquid in use from said flow regulation means (30) to said delivery site, said flow regulation means (30) comprising first and second (32, 34) enclosures isolated one from the other by means of a flexible liquid-impervious diaphragm (36) displaceable in response to pressure differential between said first and second enclosures to control liquid flow rate into said outlet means (42) from said flow regulation means, characterized in that said connection means (22) and said outlet means (42) communicate only with said first flow regulation means enclosure (32) and means is provided to establish in said second flow regulation means enclosure (34) a pressure active to displace said diaphragm (36) and variable responsively to causes of variation in the instantaneous pressure of said pressure medium in said pressure-operable supply means (10) so that said pressures are substantially matched and the diaphragm (36) is displaced to provide stabilized rate of delivery of said drug-containing liquid.

## Patentansprüche

1. Implantierbares Arzneimittelabgabesystem, umfassend eine durch Druck betätigbare Arzneimittelabgabevorrichtung (10) mit einem abgedichteten Gehäuse (12), einen ein Fluid enthaltenden anpassungsfähigen Arzneimittelvorratsbehälter (14), einen Auslaß (22) aus dem besagten Arzneimittelvorratsbehälter (14) und eine Treibmittelkammer (16), um das besagte Fluid beim Gebrauch des Systems aus dem besagten Vorratsbehälter (14) in den besagten Auslaß (22) zu drücken, eine Durchflußregelvorrichtung (30) mit einer Reglerkammer (32) und einer Meßkammer (34), wobei die besagte Reglerkammer (32) und die besagte Meßkammer (34) durch eine flexible Membran (36) voneinander getrennt und in Isolation voneinander gehalten werden, wobei der besagte Auslaß (22) aus dem besagten Arzneimittelvorratsbehälter (14) in Fluidverbindung mit der besagten Durchflußregelvorrichtung (30) steht, und die besagte Regelvorrichtung (30) einen Auslaßkatheter (42) aufweist, der die besagte Reglerkammer im Gebrauch mit einem Arzneimittelabgabeort verbindet, dadurch gekennzeichnet, daß der besagte Auslaß (22) das besagte Fluid nur der besagten Reglerkammer (32) zuführen kann, und eine Einrichtung vorgesehen ist, um in der besagten Meßkammer (34) einen Vergleichsdruck herzustellen, der dem Druck in der besagten Treibmittelkammer im wesentlichen gleich ist.

2. System nach Anspruch 1, bei welchem die besagte Einrichtung zur Herstellung eines Vergleichsdrucks eine Quelle von Treibmittel in der besagten Meßkammer (34) umfaßt, welches dasselbe wie das Treibmittel in der Treibmittelkammer (16) der besagten Arzneimittelabgabevorrichtung (10) ist.

3. System nach Anspruch 1 oder Anspruch 2, bei welchem die besagte Einrichtung zur Herstellung eines Vergleichsdrucks eine Fluidverbindung (44) zwischen der besagten Treibmittelkammer (16) und der besagten Meßkammer (34) umfaßt.

4. System nach Anspruch 2 oder Anspruch 3 und umfassend eine Drossel (40) in dem besagten Auslaßkatheter (42).

5. System nach einem beliebigen vorangehenden Anspruch und umfassend ein mit dem besagten Arzneimittelvorratsbehälter (14) verbundenes Eindringseptum (20) zum Nachfüllen des besagten Fluids.

6. System nach einem beliebigen vorangehenden Anspruch und umfassend einen Bakterienfilter (24) in der besagten Auslaßleitung (22) und einen Durchflußbegrenzer (26), der stromaufwärts von der besagten Reglerkammer (32) in der besagten Auslaßleitung angeordnet ist.

7. System nach einem beliebigen vorangehenden Anspruch und umfassend eine Reglerdichtung (38) in der besagten Regelkammer (32) der besagten Durchflußregelvorrichtung (30), wobei die besagte Reglerdichtung (38) zum Verändern des Fluidstroms zu dem besagten Auslaßkatheter (42) in Bezug zu der besagten Membran (36) angeordnet ist.

8. System nach Anspruch 7, bei welchem die besagte flexible Membran (36) vorgespannt ist, so daß sie sich in Richtung der besagten Reglerdichtung (38) durchbiegt.

9. System nach einem beliebigen vorangehenden Anspruch, bei dem der besagte anpassungsfähige Arzneimittelvorratsbehälter (14) einen Balg (18) umfaßt, der in dem besagten abgedichteten Gehäuse (12) angeordnet ist.

10. Implantierbares Arzneimittelabgabesystem zur Abgabe einer Arzneimittel enthaltenden Flüssigkeit an einen Arzneimittelabgabeort, wobei dieses System umfaßt: eine durch Druck betätigbare Versorgungseinrichtung (10), umfassend eine Einrichtung (14), die einen kompressiblen Vorratsbehälter zur Aufnahme einer Charge der besagten Flüssigkeit und einen Druckmedium enthaltenden Einschluß (16) bildet, der angeordnet ist, um die besagte kompressible, den Vorratsbehälter bildende Einrichtung (14) mit dem besagten Druckmedium zu beaufschlagen, um im Gebrauch Arzneimittel enthaltende Flüssigkeit aus dem besagten Vorratsbehälter (14) zu treiben, eine Durchflußregeleinrichtung (30) für Arzneimittel enthaltende Flüssigkeit zur Aufnahme der besagten Flüssigkeit, Verbindungseinrichtungen (22), die eine Verbindung für einen Flüssigkeitsstrom zwischen dem besagten Vorratsbehälter (14) und der besagten Durchflußregeleinrichtung (30) bereitstellen, sowie eine Auslaßeinrichtung (42), um im Gebrauch Arzneimittel enthaltende Flüssigkeit aus der besagten Durchflußregeleinrichtung (30) an den besagten Abgabeort zu befördern, wobei die besagte Durchflußregeleinrichtung (30) einen ersten und zweiten (32, 34) Einschluß umfaßt, die mit Hilfe einer flexiblen flüssigkeitsundurchlässigen Membran (36) voneinander getrennt sind, welche ansprechend auf eine Druckdifferenz zwischen dem besagten ersten und zweiten Einschluß verlagerbar ist, um eine Durchflußmenge der Flüssigkeit aus der besagten Durchflußregeleinrichtung in die besagte Auslaßeinrichtung (42) zu steuern, dadurch gekennzeichnet, daß die besagte Verbindungseinrichtung (22) und die besagte Auslaßeinrichtung (42) nur mit dem besagten ersten Einschluß (32) der Durchflußregeleinrichtung in Verbindung stehen, und eine Einrichtung vorgesehen ist, um in dem besagten zweiten Einschluß (34) der Durchflußregeleinrichtung einen Druck herzustellen, der zum Verlagern der besagten Membran (36) wirksam und ansprechend auf Ursachen einer Veränderung des momentanen Drucks des besagten Druckmediums in der besagten durch Druck betätigbaren Versorgungseinrichtung (10) veränderlich ist, so daß die besagten Drücke im wesentlichen aneinander angepaßt sind, und die Membran (36) verlagert wird, um eine stabilisierte Abgabemenge der besagten Arzneimittel enthaltenden Flüssigkeit zu liefern.

## Revendications

1. Système implantable de distribution de médicament, comprenant un dispositif (10) de distribution de médicament actionné par une pression et comprenant un logement étanche (12), un réservoir souple (14) de médicament qui contient un fluide, une sortie (22) dudit réservoir à médicament (14) et une chambre à propulseur (16) destinée à refouler ledit fluide dudit réservoir (14) dans ladite sortie (22) lorsque le système est en service, un dispositif (30) de réglage de la circulation comprenant une chambre régulatrice (32) et une chambre détectrice (34), ladite chambre régulatrice (32) et ladite chambre détectrice (34) étant séparées et maintenues isolées l'une de l'autre par un diaphragme souple (36), ladite sortie (22) dudit réservoir à médicament (14) étant en communication fluidique avec ledit dispositif (30) de réglage de la circulation et ledit dispositif de réglage (30) comportant un cathéter de sortie (42) qui raccorde en service ladite chambre régulatrice à un site de distribution de médicament, caractérisé en ce que ladite sortie (22) ne peut envoyer ledit fluide qu'à ladite chambre régulatrice (22) et un moyen est prévu pour établir dans ladite chambre détectrice (34) une pression de référence qui est sensiblement égale à la pression régnant dans ladite chambre à propulseur.

2. Système selon la revendication 1, dans lequel ledit moyen pour établir une pression de référence comprend dans ladite chambre détectrice (34) une source de propulseur qui est le même que le propulseur se trouvant dans ladite chambre (16) à propulseur dudit dispositif (10) de distribution de médicament.

3. Système selon la revendication 1 ou la revendication 2, dans lequel ledit moyen pour établir une pression de référence consiste en un raccord fluidique (44) entre ladite chambre à propulseur (16) et ladite chambre détectrice (34).

4. Système selon la revendication 2 ou la revendication 3, et comprenant un étranglement (40) dans ledit cathéter de sortie (42).

5. Système selon l'une quelconque des revendications précédentes et comprenant un septum de pénétration (20) relié audit réservoir à médicament (14) pour le réapprovisionnement en ledit fluide.

6. Système selon l'une quelconque des revendications précédentes et comprenant un filtre bactériel (24) dans ledit conduit de sortie (22) et un étranglement de la circulation (26) disposé dans ledit conduit de sortie en amont de ladite chambre régulatrice (32).

7. Système selon l'une quelconque des revendications précédentes et comprenant un joint régulateur (38) dans ladite chambre de réglage (32) dudit dispositif (30) de réglage de la circulation, ledit joint régulateur (38) étant disposé par rapport audit diaphragme (36) de manière à faire varier la circulation du fluide vers ledit cathéter de sortie (42).

8. Système selon la revendication 7, dans lequel ledit diaphragme souple (36) est soumis à une force le faisant fléchir vers ledit joint régulateur (38).

9. Système selon l'une quelconque des revendications précédentes, dans lequel ledit réservoir souple de médicament (14) consiste en un soufflet (18) disposé dans ledit logement étanche (12).

10. Système de distribution de médicament pour envoyer un liquide contenant un médicament à un site de distribution de médicament, ledit système comprenant un moyen d'alimentation (10) commandé par pression et comprenant un moyen constituant un réservoir compressible (14) destiné à contenir une charge dudit liquide et un logement (16) contenant un fluide sous pression et conçu pour faire agir ledit fluide sous pression contre ledit moyen (14) constituant un réservoir compressible afin de refouler en service le liquide contenant le médicament hors dudit réservoir (14), un moyen (30) de réglage de la circulation du liquide contenant le médicament étant destiné à recevoir ledit liquide, un moyen de raccord (22) établissant une communication de la circulation de liquide entre ledit réservoir (14) et ledit moyen de réglage de la circulation (30) et un moyen de sortie (42) étant destiné à transférer en service le liquide contenant le médicament dudit moyen de réglage de la circulation (30) au site de distribution, ledit moyen de réglage de la circulation (30) comprenant des premier et second logements (32, 34) isolés l'un de l'autre par un diaphragme souple (36) imperméable aux liquides et déplaçable en réponse à un différentiel de pression entre lesdits premier et second logements pour commander le débit du liquide dudit moyen de réglage de la circulation dans ledit moyen de sortie (42), caractérisé en ce que ledit moyen de raccord

(22) et ledit moyen de sortie (42) ne communiquent qu'avec ledit premier logement (32) du moyen de réglage de la circulation et un moyen est prévu pour établir dans ledit second logement (34) du moyen de réglage de la circulation une pression agissant pour déplacer ledit diaphragme (36) et variable en réponse de manière à provoquer une variation de la pression instantanée dudit fluide sous pression dans ledit moyen d'alimentation (10) commandé par pression de façon que lesdites pressions soient sensiblement équilibrées et que le diaphragme (36) soit déplacé pour produire un débit stabilisé de distribution dudit liquide contenant un médicament.
